# EUROPEAN PATENT SPECIFICATION

(11) **EP 1 003 872 B1**
(45) Date of publication and mention of the grant of the patent: **22.10.2003**
(21) Application number: 98934566.5
(22) Date of filing: 14.07.1998
(51) Int. Cl.: C12N 15/31, C07K 14/32, C12N 15/62, C12N 15/75, C12N 1/21

(54) **INCREASING PRODUCTION OF PROTEINS IN GRAM-POSITIVE MICROORGANISMS**
ERHÖHUNG DER PROTEINPRODUKTION IN GRAM-POSITIVEN MIKROORGANISMEN
AUGMENTATION DE LA PRODUCTION DE PROTEINES DANS DES MICRO-ORGANISMES GRAM-POSITIFS

(30) Priority: 15.07.1997 EP 97305228
(43) Date of publication of application: 31.05.2000
(73) Proprietor: GENENCOR INTERNATIONAL, INC., Palo Alto, California 94304 (US)
(72) Inventor: QUAX, Wilhelmus, J., NL-2253 VB Voorschoten (NL); CALDWELL, Robert, M., Belmont, CA 94002 (US)
(74) Representative: Kiddle, Simon John
(86) International application number: US9814648
(87) International publication number: WO99004006

(56) References cited:
- WO-A-94/19471
- NISHIYAMA K ET AL: "A novel membrane protein involved in protein translocation across the cytoplasmic membrane of Escherichia coli" EMBO JOURNAL, vol. 12, no. 9, 1993, pages 3409-3415, XP002084809
- SUH J -W ET AL: "ISOLATION OF A SECY HOMOLOGUE FROM BACILLUS SUBTILIS: EVIDENCE FOR A COMMON PROTEIN EXPORT PATHWAY IN EUBACTERIA" MOLECULAR MICROBIOLOGY, vol. 4, no. 2, 1 January 1990, pages 305-314, XP000607144

## Description

### FIELD OF THE INVENTION

The present invention generally relates to expression of proteins in gram-positive microorganisms and specifically to the gram positive microorganism secretion factor, SecG. The present invention also provides expression vectors, methods and systems for the production of proteins in gram-positive microorganisms.

### BACKGROUND OF THE INVENTION

Gram-positive microorganisms, such as members of the group *Bacillus,* have been used for large-scale industrial fermentation due, in part, to their ability to secrete their fermentation products into the culture media. In gram-positive bacteria, secreted proteins are exported across a cell membrane and a cell wall, and then are subsequently released into the external media usually obtaining their native conformation.

Secretion factors from Gram-positive microorganisms which have been identified and reported in the literature include SecA (Sadaie Y., Takamatsu h., Nakamura k., Yamane k.; Gene 98:101-105, 1991)., SecY (Suh J.-W., Boylan S.A., Thomas S.M., Dolan K.M., Oliver D.B., Price C.W.; Mol. Microbiol. 4:305-314, 1990)., SecE (Jeong S., Yoshikawa H., Takahashi H.; Mol. Microbiol. 10:133-142, 1993), FtsY an FfH (WO97/03197, PCT/NL 96/00278), and PrsA (WO 94/19471).

By contrast, in the gram-negative microorganism, E.coli, protein is transported to the periplasm rather than across the cell membrane and cell wall and into the culture media. E.coli has at least two types of components of the secretory mechanism, soluble cytoplasmic proteins and membrane associated proteins. Reported E.coli secretion factors include the soluble cytoplasmic proteins, SecB and heat shock proteins; the peripheral membrane-associated protein SecA; and the integral membrane proteins SecY, SecE, SecD and SecF.

In spite of advances in understanding portions of the protein secretion machinery in procaryotic cells, the complete mechanism of protein secretion, especially for gram-positive microorganisms, such as *Bacillus,* has yet to be fully elucidated.

### SUMMARY OF THE INVENTION

The capacity of the secretion machinery of a Gram-positive microorganism may become a limiting factor or bottleneck to protein secretion and the production of proteins in secreted form, in particular when the proteins are recombinantly introduced and overexpressed by the host cell. The present invention provides a means for alleviating that bottle neck.

The present invention is based, in part, upon the discovery of a Bacillus subtilis SecG secretion factor (also referred to herein as YVAL) identified in heretofore uncharacterised translated genomic DNA by its homology with a consensus sequence for SecG (based upon SecG sequences for Escherichia, Haemophilus, and Mycoplasma organisms) and the demonstration that B. subtilis SecG is a functional homolog of E.coli Sec G. The present invention is also based, in part, upon the discovery that B.subtilis SecG in combination with other B.subtilis secretion factors forms a functional preprotein translocase.

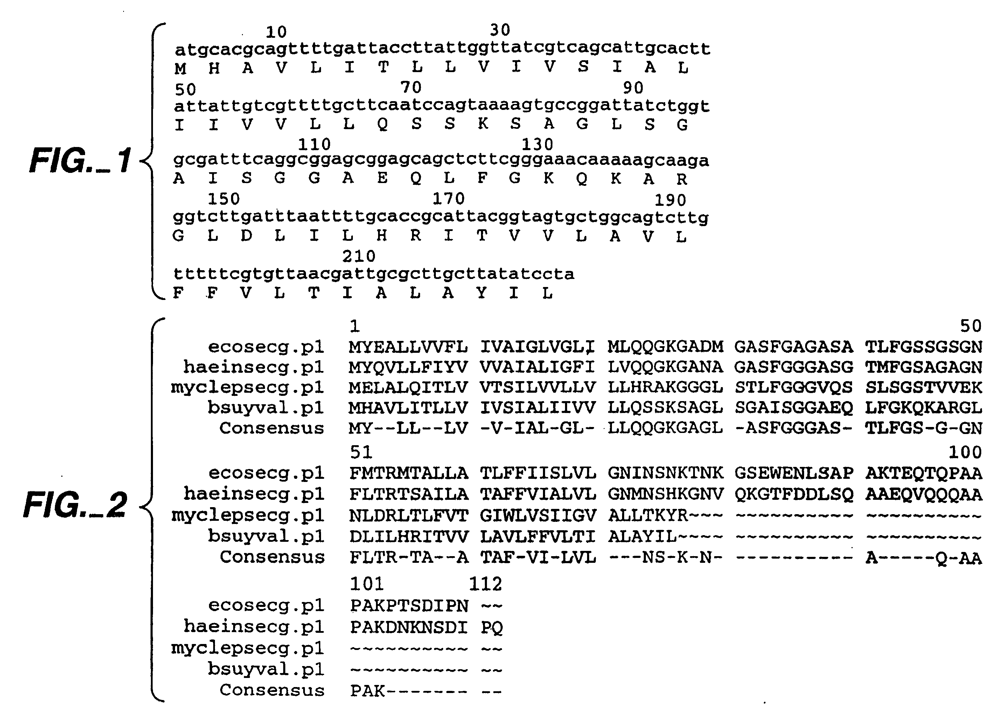

The present invention provides isolated nucleic acid and deduced amino acid sequences for *B. subtilis* SecG. The amino acid sequence for *B. subtilis* SecG is shown in Figure 1. The nucleic acid sequence encoding *B. subtilis* SecG is shown in Figure 1.

The present invention also provides improved methods for secreting proteins from gram-positive microorganisms. Accordingly, the present invention provides an improved method for secreting a desired protein in a gram-positive microorganism comprising the steps of obtaining a gram-positive microorganism host cell comprising nucleic acid encoding SecG which has at least 80% amino acid sequence identity to B. subtilis SecG set out in Figure 1 and is capable of modulating secretion in a gram-positive microorganism, wherein said nucleic acid is under the control of expression signals capable of expressing SecG in a gram-positive microorganism said microorganism further comprising nucleic acid encoding said protein; and culturing said microorganism under conditions suitable for expression of SecG and expression and secretion of said protein. In one embodiment of the present invention, the desired protein is homologous or naturally occurring in the gram-positive microorganism. In another embodiment of the present invention, the desired protein is heterologous to the gram-positive microorganism.

In one aspect of the present invention, a microorganism is genetically engineered to produce a desired protein, such as an enzyme, growth factor or hormone. The enzyme is selected from the group consisting of proteases, carbohydrases including amylases, cellulases, xylanases, and lipases; isomerases such as racemases, epimerases, tautomerases, or mutases; transferases, kinases and phophatases acylases, amidases, esterases, reductases, oxidases. In a further embodiment the expression of the secretion factor SecG is coordinated with the expression of other components of the secretion machinery. Preferably other components of the secretion machinary, i.e., translocase, SecA, SecY, SecE and/or other secretion known to those of skill in the art are modulated in expression at an optimal ratio to SecG. For example, it may be desired to overexpress multiple secretion factors in addition to SecG for optimum enhancement of the secretion machinary. In one particular embodiment disclosed herein, B.subtilis SecG is expressed along with B.subtilis SecYE and SecA to form a functional preprotein translocase.

### BRIEF DESCRIPTION OF THE DRAWINGS

Figure 1 shows the nucleic acid sequence for *secG* and the amino acid sequence of SecG.
Figure 2 shows an amino acid alignment of the SecG sequence from E.coli (ecosecg.p1). Haemophilus (haeinsecg.p1). Mycoplasma (myclepsecg.p1) and B.subtilis (bsuyval.p1) and the SecG consensus sequence of the four organisms.

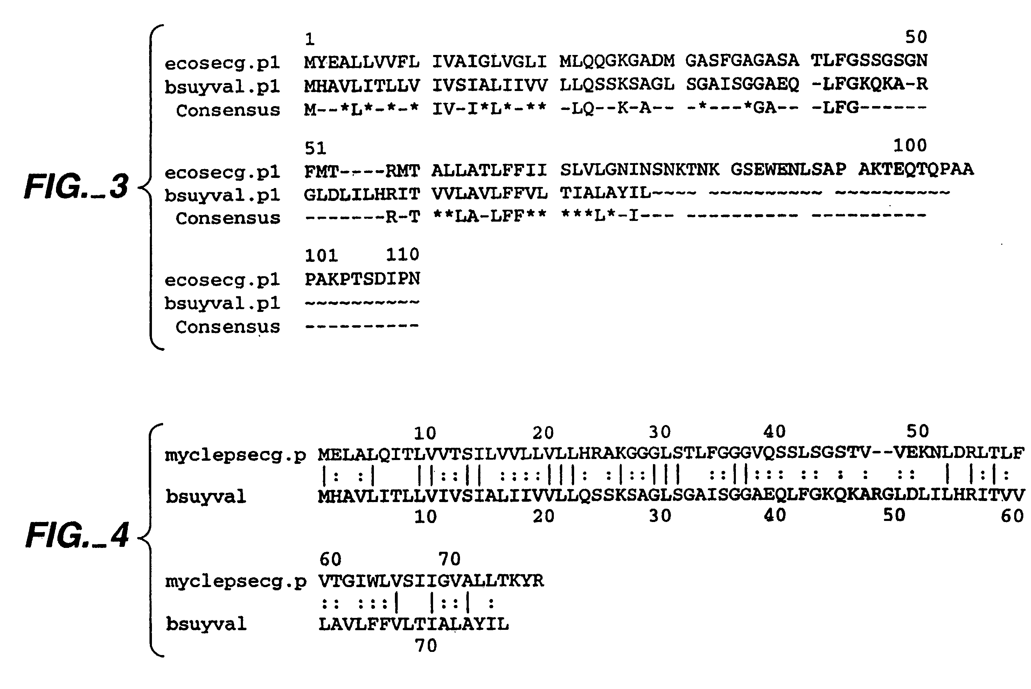

Figure 3 shows the amino acid identity between B.subtilis SecG and E.coli SecG.
Figure 4 shows the amino acid identity between B.subtilis SecG and Mycoplasma SecG.
Figure 5 shows a hydrophilicity profile of B subtilis SecG.
Figures 6A-6B. Figure 6A shows a commassie stained SDS-PAGE of cell fractions of B.subtilis DB104 and DB104:ΔyvaL. Lower case "c" refers to cellular fraction; lower case "m" refers to medium. The position of a polypeptide band is indicated that is present in the wild-type cells but absent in the deletion mutant.
Figure 6B shows the proteinase K digestion of cell associated proteins. The digestion of the polypeptide band at 30 kDa is absent in the DB104: ΔyvaL cells. The final lane shows a control with triton X-100 to demonstrate that proteinase K is present in excess amounts.
Figures 7A-7C.

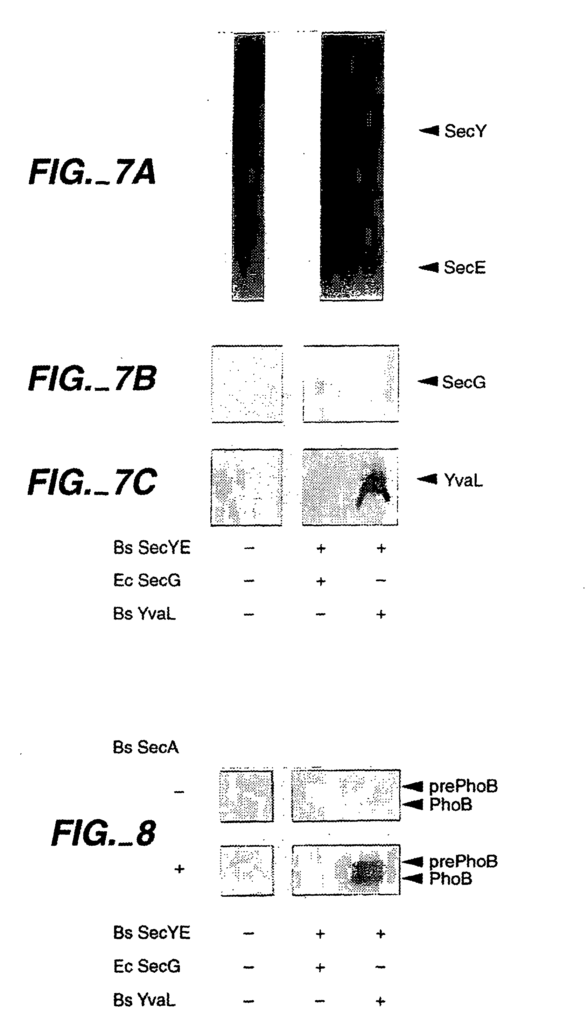

Figure 7A shows a commassie stained SDS-PAGE of E.coli inner membrane vesicles expressing the B.subtilis SecYE and either E.coli SecG or B.subtilis SecG (YvaL) compared to wild type vesicles. The position of B.subtilis SecY and SecE is indicated.
Figure 7B shows an immunoblot developed with a pAb directed against a synthetic polypeptide of E.coli SecG.
Figure 7C shows an immunoblot developed with a pAb directed against a synthetic polypeptide of B.subtilis SecG.
Figure 8 shows an In vitro translocation of ¹²⁵I-labelled prePhoB into E.coli inside out vesicles. Vesicles were stripped for SecA and purified B.subtilis SecA was added when indicated.

### DETAILED DESCRIPTION

### Definitions

As used herein, the genus *Bacillus* includes all members known to those of skill in the art, including but not limited to *B. subtilis, B. licheniformis, B. lentus, B. brevis, B. stearothermophilus, B. alkalophilus, B. amyloliquefaciens, B. coagulans, B. circulans, B. lautus* and *B. thuringiensis*.

The present invention encompasses novel SecG secretion factors from gram positive microorganism. In a preferred embodiment, the gram-positive organism is *Bacillus.* In another preferred embodiment, the gram-positive organism is *B. subtilis*. As used herein, the phrase, *"B.subtilis* SecG secretion factor" refers to the deduced amino acid sequence shown in Figure 1. The present invention encompasses variants of the amino acid sequence disclosed in Figure 1 that are able to modulate secretion alone or in combination with other secretions factors.

As used herein, "nucleic acid" refers to a nucleotide or polynucleotide sequence, and fragments or portions thereof, and to DNA or RNA of genomic or synthetic origin which may be double-stranded or single-stranded, whether representing the sense or antisense strand. As used herein "amino acid" refers to peptide or protein sequences or portions thereof. As used herein, lower case "*secG*" is used to designate a nucleic acid sequence, whereas upper case "SecG" is used to designate an amino acid sequence. A *"B.subtilis* polynucleotide homolog" or "polynucleotide homolog" as used herein refers to a novel polynucleotide that has at least 80%, at least 90% and at least 95% identity to the *secG* polynucleotide in Figure 1 or which is capable of hybridizing to the polynucleotide of Figure 1 under conditions of high stringency and which encodes an amino acid sequence that is able to modulate secretion of the gram-positive microorganism from which it is derived. Modulate as used herein refers to the ability of a secretion factor to alter the secretion patterns of proteins.

The terms "isolated" or "purified" as used herein refer to a nucleic acid or amino acid that is removed from at least one component with which it is naturally associated.

As used herein, the term "heterologous protein" refers to a protein or polypeptide that does not naturally occur in a gram-positive host cell. Examples of heterologous proteins include enzymes such as hydrolases including proteases, cellulases, amylases, other carbohydrases, and lipases; isomerases such as racemases, epimerases, tautomerases, or mutases; transferases, kinases and phophatases. The heterologous gene may encode therapeutically significant proteins or peptides, such as growth factors, cytokines, ligands, receptors and inhibitors, as well as vaccines and antibodies. The gene may encode commercially important industrial proteins or peptides, such as proteases, carbohydrases such as amylases and glucoamylases, cellulases, oxidases and lipases. The gene of interest may be a naturally occurring gene, a mutated gene or a synthetic gene.

The term "homologous protein" refers to a protein or polypeptide native or naturally occurring in a gram-positive host cell. The invention includes host cells producing the homologous protein via recombinant DNA technology. The present invention encompasses a gram-positive host cell having a deletion or interruption of the nucleic acid encoding the naturally occurring homologous protein, such as a protease, and having nucleic acid encoding the homologous protein, or a variant thereof re-introduced in a recombinant form. In another embodiment, the host cell produces the homologous protein.

### Detailed Description of the Preferred Embodiments

The present invention provides novel gram-positive microorganism secretion factors and methods that can be used in gram-positive microorganisms to ameliorate the bottleneck to protein secretion and the production of proteins in secreted form, in particular when the proteins are recombinantly introduced and overexpressed by the host cell. The present invention provides the secretion factor SecG derived from *Bacillus subtilis.*

### I. SecG Nucleic Acid and Amino Acid Sequences

### SecG nucleic acid sequences

The *SecG* polynucleotide having the sequence as shown in Figure 1 encodes the *Bacillus subtilis* secretion factor SecG. A FASTA search of *Bacillus subtilis* translated genomic sequences with the E.coli SecG sequence alone did not identify the B. subtilis SecG. The *Bacillus subtilis* SecG was identified via a FASTA search of *Bacillus subtilis* translated genomic sequences using a consensus sequence of 30 amino acids of SecG derived from E.coli,
Haemophilus and Mycoplasma species as shown in Figure 2. The consensus sequence used was
"LVGLILLQQG KGAXXGASFG GGASXTLFGS" given in the amino terminus to carboxy terminus direction with the FASTA search (Release 1.0, released on June 11, 1997) parameters being Scoring matrix: GenRunData: blosum50.cmp; variable pamfactor used; Gap creation penalty: 12; and Gap extension penalty: 2.

The present invention provides gram-positive *secG* polynucleotides which may be used alone or together with other secretion factors, such as SecY, SecE and SecA, in a gram-positive host cell for the purpose of increasing the secretion of desired heterologous or homologous proteins or polypeptides.

The present invention encompasses *secG* polynucleotide homologs encoding novel gram-positive microorganism SecG whether encoded by one or multiple polynucleotides which have at least 80%, or at least 90% or at least 95% identity to *B. subtilis* SecG as long as the homolog encodes a protein that is able to function by modulating secretion in a gram-positive microorganism. As will be understood by the skilled artisan, due to the degeneracy of the genetic code, a variety of polynucleotides, i.e., *SecG* polynucleotide variants, can encode the *Bacillus subtilis* secretion factors SecG. The present invention encompasses all such polynucleotides.

Gram-positive polynucleotide homologs of *B.subtilis* SecG may be obtained by standard procedures known in the art from, for example, cloned DNA (*e.g*., a DNA "library"), genomic DNA libraries, by chemical synthesis once identified, by cDNA cloning, or by the cloning of genomic DNA, or fragments thereof, purified from a desired cell. *(See,* for example, Sambrook *et al*., 1989, Molecular Cloning, A Laboratory Manual, 2d Ed., Cold Spring Harbor Laboratory Press, Cold Spring Harbor, New York; Glover, D.M. (ed.), 1985, DNA Cloning: A Practical Approach, MRL Press, Ltd., Oxford, U.K. Vol. I, II.) A preferred source is from genomic DNA. Nucleic acid sequences derived from genomic DNA may contain regulatory regions in addition to coding regions. Whatever the source, the isolated *secG* gene should be molecularly cloned into a suitable vector for propagation of the gene.

In the molecular cloning of the gene from genomic DNA, DNA fragments are generated, some of which will encode the desired gene. The DNA may be cleaved at specific sites using various restriction enzymes. Alternatively, one may use DNAse in the presence of manganese to fragment the DNA, or the DNA can be physically sheared, as for example, by sonication. The linear DNA fragments can then be separated according to size by standard techniques, including but not limited to, agarose and polyacrylamide gel electrophoresis and column chromatography.

Once the DNA fragments are generated, identification of the specific DNA fragment containing the SecG may be accomplished in a number of ways. For example, a *B.subtilis* SecG gene of the present invention or its specific RNA, or a fragment thereof, such as a probe or primer, may be isolated and labeled and then used in hybridization assays to detect a gram-positive SecG gene. (Benton, W. and Davis, R., 1977, Science 196:180; Grunstein, M. And Hogness, D., 1975, Proc. Natl. Acad. Sci. USA 72:3961). Those DNA fragments sharing substantial sequence similarity to the probe will hybridize under stringent conditions.

Accordingly, the present invention provides a method for the detection of gram-positive SecG polynucleotide homologs which comprises hybridizing part or all of a nucleic acid sequence of *B. subtilis* SecG with gram-positive microorganism nucleic acid of either genomic or cDNA origin.

Also included within the scope of the present invention are gram-positive microorganism polynucleotide sequences that are capable of hybridizing to the nucleotide sequence of *B.subtilis* SecG under conditions of intermediate to maximal stringency. Hybridization conditions are based on the melting temperature (Tm) of the nucleic acid binding complex, as taught in Berger and Kimmel (1987, Guide to Molecular Cloning Techniques. Methods in Enzymology. Vol 152. Academic Press, San Diego CA) incorporated herein by reference, and confer a defined "stringency" as explained below.

Also included within the scope of the present invention are novel gram-positive microorganism *secG* polynucleotide sequences that are capable of hybridizing to part or all of the *secG* nucleotide sequence of Figure 1 under conditions of intermediate to maximal stringency. Hybridization conditions are based on the melting temperature (Tm) of the nucleic acid binding complex, as taught in Berger and Kimmel (1987, Guide to Molecular Cloning Techniques, Methods in Enzymology, Vol 152, Academic Press, San Diego CA) incorporated herein by reference, and confer a defined "stringency" as explained below.

"Maximum stringency" typically occurs at about Tm-5°C (5°C below the Tm of the probe); "high stringency" at about 5°C to 10°C below Tm; "intermediate stringency" at about 10°C to 20°C below Tm; and "low stringency" at about 20°C to 25°C below Tm. As will be understood by those of skill in the art, a maximum stringency hybridization can be used to identify or detect identical polynucleotide sequences while an intermediate or low stringency hybridization can be used to identify or detect polynucleotide sequence homologs.

The term "hybridization" as used herein shall include "the process by which a strand of nucleic acid joins with a complementary strand through base pairing" (Coombs J (1994) Dictionary of Biotechnology, Stockton Press, New York NY). The process of amplification as carried out in polymerase chain reaction (PCR) technologies is described in Dieffenbach CW and GS Dveksler (1995, PCR Primer, a Laboratory Manual, Cold Spring Harbor Press, Plainview NY). A nucleic acid sequence of at least about 10 nucleotides and as many as about 60 nucleotides from the SecG nucleotide sequence of Figure 1, preferably about 12 to 30 nucleotides, and more preferably about 20-25 nucleotides can be used as a probe or PCR primer.

### Amino Acid Sequences

The *B. subtilis secG* polynucleotide as shown in Figure 1 encodes *B. subtilis* SecG. The present invention encompasses novel gram positive microorganism amino acid variants of the amino acid sequence shown in Figure 1 that are at least 80% identical, at least 90% identical and at least 95% identical to the sequence shown in Figure 1 as long as the amino acid sequence variant is able to function by modulating secretion of proteins in gram-positive microorganisms alone or in combination with other secretion factors.

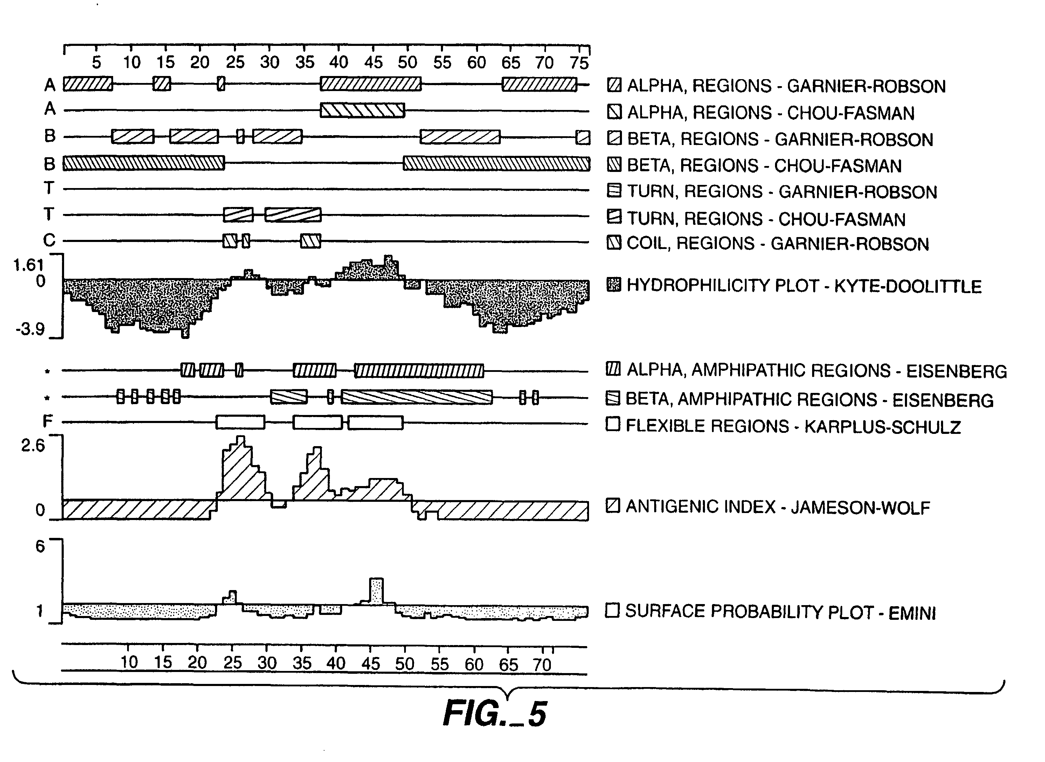

The secretion factor SecG as shown in Figure 1 was subjected to a FASTA (Lipmann Pearson routine) amino acid search against a consensus amino acid sequence for SecG. The amino acid alignment is shown in Figure 2. The hydrophilicity profile for B.subtilis SecG as shown in Figure 5 shows two potential membrane spanning regions.

### II. Expression Systems

The present invention provides expression systems for the enhanced production and secretion of desired heterologous or homologous proteins in gram-positive microorganisms.

### a. Coding Sequences

In the present invention, the vector comprises at least one copy of nucleic acid encoding a gram-positive microorganism SecG secretion factor and preferably comprises multiple copies. In a preferred embodiment, the gram-positive microorganism is *Bacillus.* In another preferred embodiment, the gram-positive microorganism is *Bacillus subtilis.* In a preferred embodiment, polynucleotides which encode *B. subtilis* SecG, or fragments thereof, or fusion proteins or polynucleotide homolog sequences that encode amino acid variants of SecG, may be used to generate recombinant DNA molecules that direct the expression of SecG, or amino acid variants thereof, respectively, in gram-positive host cells. In a preferred embodiment, the host cell belongs to the genus *Bacillus.* In another preferred embodiment, the host cell is *B. subtilis*.

As will be understood by those of skill in the art, it may be advantageous to produce polynucleotide sequences possessing non-naturally occurring codons. Codons preferred by a particular gram-positive host cell (Murray E et al (1989) Nuc Acids Res 17:477-508) can be selected, for example, to increase the rate of expression or to produce recombinant RNA transcripts having desirable properties, such as a longer half-life, than transcripts produced from naturally occurring sequence.

Altered gram positive *secG* polynucleotide sequences which may be used in accordance with the invention include deletions, insertions or substitutions of different nucleotide residues resulting in a polynucleotide that encodes the same or a functionally equivalent *secG* homolog, respectively. As used herein a "deletion" is defined as a change in either nucleotide or amino acid sequence in which one or more nucleotides or amino acid residues, respectively, are absent.

As used herein an "insertion" or "addition" is that change in a nucleotide or amino acid sequence which has resulted in the addition of one or more nucleotides or amino acid residues, respectively, as compared to the naturally occurring gram positive *secG.*

As used herein "substitution" results from the replacement of one or more nucleotides or amino acids by different nucleotides or amino acids, respectively.

The encoded protein may also show deletions, insertions or substitutions of amino acid residues which produce a silent change and result in a functionally equivalent gram-positive secG variant. Deliberate amino acid substitutions may be made on the basis of similarity in polarity, charge, solubility, hydrophobicity, hydrophilicity, and/or the amphipathic nature of the residues as long as the variant retains the ability to modulate secretion. For example, negatively charged amino acids include aspartic acid and glutamic acid; positively charged amino acids include lysine and arginine; and amino acids with uncharged polar head groups having similar hydrophilicity values include leucine, isoleucine, valine; glycine, alanine; asparagine, glutamine; serine, threonine, phenylalanine, and tyrosine.

The *secG* polynucleotides of the present invention may be engineered in order to modify the cloning, processing and/or expression of the gene product. For example, mutations may be introduced using techniques which are well known in the art, eg, site-directed mutagenesis to insert new restriction sites, to alter glycosylation patterns or to change codon preference, for example.

In one embodiment of the present invention, a *secG* polynucleotide may be ligated to a heterologous sequence to encode a fusion protein. A fusion protein may also be engineered to contain a cleavage site located between the *SecG* nucleotide sequence and the heterologous protein sequence, so that the SecG protein may be cleaved and purified away from the heterologous moiety.

### b. Vector Sequences

Expression vectors used in expressing the secretion factors of the present invention in gram-positive microorganisms comprise at least one promoter associated with a gram-positive SecG, which promoter is functional in the host cell. In one embodiment of the present invention, the promoter is the wild-type promoter for the selected secretion factor and in another embodiment of the present invention, the promoter is heterologous to the secretion factor, but still functional in the host cell.

Additional promoters associated with heterologous nucleic acid encoding desired proteins or polypeptides may be introduced via recombinant DNA techniques. In one embodiment of the present invention, the host cell is capable of overexpressing a heterologous protein or polypeptide and nucleic acid encoding one or more secretion factor(s) is(are) recombinantly introduced. In one preferred embodiment of the present invention, nucleic acid encoding SecG is stably integrated into the microorganism genome. In another embodiment, the host cell is engineered to overexpress a secretion factor of the present invention and nucleic acid encoding the heterologous protein or polypeptide is introduced via recombinant DNA techniques. Example III demonstrates that B.subtilis SecG can be overexpressed in a host cell. The present invention encompasses gram-positive host cells that are capable of overexpressing other secretion factors known to those of skill in the art, including but not limited to, SecA, SecY, SecE or other secretion factors known to those of skill in the art or identified in the future. In an embodiment disclosed herein in Example II, it is demonstrated that B.subtilis SecG along with B.subtilis secretion factors SecY, E, and A, is able to participate in forming a functional preprotein translocase.

In a preferred embodiment, the expression vector contains a multiple cloning site cassette which preferably comprises at least one restriction endonuclease site unique to the vector, to facilitate ease of nucleic acid manipulation. In a preferred embodiment, the vector also comprises one or more selectable markers. As used herein, the term selectable marker refers to a gene capable of expression in the gram-positive host which allows for ease of selection of those hosts containing the vector. Examples of such selectable markers include but are not limited to antibiotics, such as, erythromycin, actinomycin, chloramphenicol and tetracycline.

### c. Transformation

In one embodiment of the present invention, nucleic acid encoding one or more gram-positive secretion factor(s) of the present invention is introduced into a gram-positive host cell via an expression vector capable of replicating within the host cell. Suitable replicating plasmids for *Bacillus* are described in Molecular Biological Methods for *Bacillus,* Ed. Harwood and Cutting, John Wiley & Sons, 1990, hereby expressly incorporated by reference; see chapter 3 on plasmids. Suitable replicating plasmids for *B. subtilis* are listed on page 92.

In another embodiment, nucleic acid encoding a gram-positive micro-organism SecG stably integrated into the microorganism genome. Preferred gram-positive host cells are from the genus *Bacillus.* Another preferred gram-positive host cell is *B. subtilis.* Several strategies have been described in the literature for the direct cloning of DNA in *Bacillus.* Plasmid marker rescue transformation involves the uptake of a donor plasmid by competent cells carrying a partially homologous resident plasmid (Contente *et al., Plasmid 2*:555-571 (1979); Haima *et al., Mol. Gen. Genet. 223*:185-191 (1990); Weinrauch *et al., J. Bacteriol. 154(3)*:1077-1087 (1983); and Weinrauch *et al., J. Bacteriol. 169(3)*:1205-1211 (1987)). The incoming donor plasmid recombines with the homologous region of the resident "helper" plasmid in a process that mimics chromosomal transformation.

Transformation by protoplast transformation is described for *B. subtilis* in Chang and Cohen, (1979) Mol. Gen. Genet 168:111-115; for B.megaterium in Vorobjeva et al., (1980) FEMS Microbiol. Letters 7:261-263; for B. amyloliquefaciens in Smith et al., (1986) Appl. and Env. Microbiol. 51:634; for B.thuringiensis in Fisher et al., (1981) Arch. Microbiol. 139:213-217; for B.sphaericus in McDonald (1984) J. Gen. Microbiol. 130:203; and B.larvae in Bakhiet et al., (1985) 49:577. Mann et al., (1986, Current Microbiol. 13:131-135) report on transformation of *Bacillus* protoplasts and Holubova, (1985) Folia Microbiol. 30:97) disclose methods for introducing DNA into protoplasts using DNA containing liposomes.

### III. Identification of Transformants

Although the presence/absence of marker gene expression suggests that the gene of interest is also present, its presence and expression should be confirmed. For example, if the nucleic acid encoding SecG is inserted within a marker gene sequence, recombinant cells containing the insert can be identified by the absence of marker gene function. Alternatively, a marker gene can be placed in tandem with nucleic acid encoding the secretion factor under the control of a single promoter. Expression of the marker gene in response to induction or selection usually indicates expression of the secretion factor as well.

Alternatively, host cells which contain the coding sequence for a secretion factor and express the protein may be identified by a variety of procedures known to those of skill in the art. These procedures include, but are not limited to, DNA-DNA or DNA-RNA hybridization and protein bioassay or immunoassay techniques which include membrane-based, solution-based, or chip-based technologies for the detection and/or quantification of the nucleic acid or protein.

The presence of the *secG* polynucleotide sequence can be detected by DNA-DNA or DNA-RNA hybridization or amplification using probes, portions or fragments derived from the B.subtilis *secG* polynucleotide.

### IV. Secretion Assays

In an embodiment disclosed herein in Example IV, it is demonstrated that a B.subtilis microorganism having a disruption in nucleic acid encoding SecG appears to be defective in the secretion of some extracellular proteins.

Means for determining the levels of secretion of a heterologous or homologous protein in a gram-positive host cell and detecting secreted proteins include, using either polyclonal or monoclonal antibodies specific for the protein. Examples include enzyme-linked immunosorbent assay (ELISA), radioimmunoassay (RIA) and fluorescent activated cell sorting (FACS). These and other assays are described, among other places, in Hampton R et al (1990, Serological Methods, a Laboratory Manual, APS Press, St Paul MN) and Maddox DE et al (1983, J Exp Med 158:1211).

A wide variety of labels and conjugation techniques are known by those skilled in the art and can be used in various nucleic and amino acid assays. Means for producing labeled hybridization or PCR probes for detecting specific polynucleotide sequences include oligolabeling, nick translation, end-labeling or PCR amplification using a labeled nucleotide. Alternatively, the nucleotide sequence, or any portion of it, may be cloned into a vector for the production of an mRNA probe. Such vectors are known in the art, are commercially available, and may be used to synthesize RNA probes in vitro by addition of an appropriate RNA polymerase such as T7, T3 or SP6 and labeled nucleotides.

A number of companies such as Pharmacia Biotech (Piscataway NJ), Promega (Madison WI), and US Biochemical Corp (Cleveland OH) supply commercial kits and protocols for these procedures. Suitable reporter molecules or labels include those radionuclides, enzymes, fluorescent, chemiluminescent, or chromogenic agents as well as substrates, cofactors, inhibitors, magnetic particles and the like. Patents teaching the use of such labels include US Patents 3,817,837; 3,850,752; 3,939,350; 3,996,345; 4,277,437; 4,275,149 and 4,366,241. Also, recombinant immunoglobulins may be produced as shown in US Patent No. 4,816,567.

### V. Purification of Proteins

Gram positive host cells transformed with polynucleotide sequences encoding heterologous or homologous protein may be cultured under conditions suitable for the expression and recovery of the encoded protein from cell culture. The protein produced by a recombinant gram-positive host cell comprising a secretion factor of the present invention will be secreted into the culture media. Other recombinant constructions may join the heterologous or homologous polynucleotide sequences to nucleotide sequence encoding a polypeptide domain which will facilitate purification of soluble proteins (Kroll DJ et al (1993) DNA Cell Biol 12:441-53).

Such purification facilitating domains include, but are not limited to, metal chelating peptides such as histidine-tryptophan modules that allow purification on immobilized metals (Porath J (1992) Protein Expr Purif 3:263-281), protein A domains that allow purification on immobilized immunoglobulin, and the domain utilized in the FLAGS extension/affinity purification system (Immunex Corp, Seattle WA). The inclusion of a cleavable linker sequence such as Factor XA or enterokinase (Invitrogen, San Diego CA) between the purification domain and the heterologous protein can be used to facilitate purification.
The manner and method of carrying out the present invention may be more fully understood by those of skill in the art by reference to the following examples, which examples are not intended in any manner to limit the scope of the present invention or of the claims directed thereto.

### Example I

Example I provides the Materials and Methods used in Examples II-VI.

### a. Bacterial strains and growth media

Strains were grown in Luria-Bertani Broth or on Luria-Bertani agar. When necessary, the medium was supplemented with relevant antibiotics as indicated. Construction of vectors was done in *E. coli* DH5α (*supE44, ΔlacU169, (φ80lacZΔM15), hsdR17, recA1, endAI, gyrA96, thi-1, re1A1)*. Chromosomal deletions and growth experiments were done in *B. subtilis* DB104 *(nprE18, aprEΔ3*) (Yang et al., 1984, Journal of Bacteriology 160:15-21).

### b. Construction of plasmids

The *E. coli secG* and *B. subtilis yvaL* genes including suitable ribosome binding sites were amplified as *Bam*HI*-XbaI* cassettes by PCR from chromosomal DNA from strains DH5α and DB104, respectively, and cloned into pBluescript SK+, the primer used are listed in Table 1. The sequences of both open reading frames were determined and compared against relevant databases. For expression in *E. coli*, the genes were cloned into pET324 (Van der Does et al., 1996) yielding pET304 (*E. coli secG*) and pET820 (*B. subtilis yvaL*).

Vectors pPR111 (a pUB110 derivative, Diderichsen et al., 1993, Plasmid 30:312-315) and pBEY13 (a gift from Dr. R. Breitlin) are shuttle vectors using a ColE1 origin for replication in *E. coli* and RepR for replication in gram-positive organisms. These plasmids encode ampicillin resistance markers for *E. coli* and phleomycin resistance markers for *B. subtilis*. Vector pBEY13 expresses the *B. subtilis secY* and *secE* genes from the constitutive staphylococcal sak promoter. Plasmids pET470 and pET471 were formed by replacing the *secYE* cassette by *E. coli secG* and *B. subtilis yvaL* respectively. Vector pAMP21 is a pGK13 (Kok et al., 1984, Applied Environmental Microbiology 48: 726-731) based broad host range vector containing the *lactococcus* derived *p32* promotoer (van der Vossen et al., 1987, Applied and Environmental Microbiology 10:2452-2457) with synthetic ribosome binding site and *Ncol* site overlapping the start codon. The *B. amyloliquefaciens* α-amylase gene was isolated by PCR from plasmid pKTH10 (Palva, 1982, Gene 1:81-87) as an *Nco*I*-Bam*HI cassette, and ligated into *Nco*I*-Bam*HI digested pAMP21. The resulting vector, named pET468, harbors the *amyQ* gene under control of the constitutive *p32* promoter. Vectors pET472 and pET473 were generated by ligating the E.coli and B. subtilis *secG* genes, respectively, containing *Bam*HI-*Bss*HII fragments from the pBluescript derivatives into BamHI-*Bss*HII-*MluI* digested pET468. Resulting vectors express *B. amyloliquefaciens* α-amylase and *secG* or *yvaL* as a tandem operon from the single *p32* promoter.

A vector for the disruption of *yvaL* was generated as follows. The regions immediately upstream and downstream of the *yvaL* were amplified from chromosomal DNA from strain DB104 as *Bam*HI*-Xba*I and *KpnI-Hinc*II cassettes respectively, and cloned into pBluescript SK+. Subsequently a *Bg*/II*-Pvu*II digested chloramphenicol resistance marker was placed between the *Bam*HI and *Hinc*II sites, yiedling pDELG2. This vector contains the chromosomal region as is present in DB104 with the *yvaL* replaced by the chloramphenicol resistance marker.

Plasmid pET812 containing a synthetic operon of *Bacillus subtilis secY, secE* and *E. coli secG,* and plasmid pET822 containing *secY*, and *secE* and *yvaL* of *B. subtilis* were constructed for expression in *E. coli* as described before (Van der Does et al., 1996) using the primers listed in Table 1.

The alkaline phosphates *phoB (phoA*III) of *B. subtilis* was amplified from chromosomal DNA of DB104 using PCR (for primers see Table 1) and N-terminally fused to a his-tag using the plasmid pET302 (van der Does et al., 1998, Biochemistry, 37: 201-210) so creating pET461. An overview of the plasmids used in this study is given in Table 2.

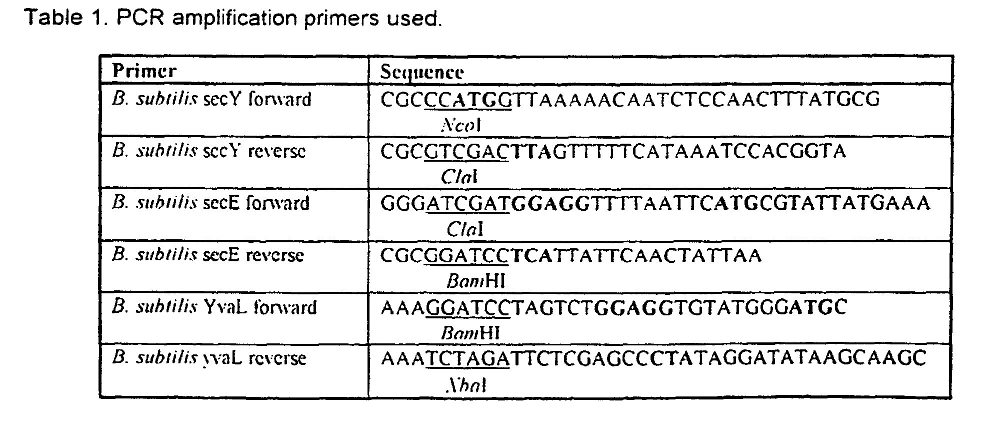

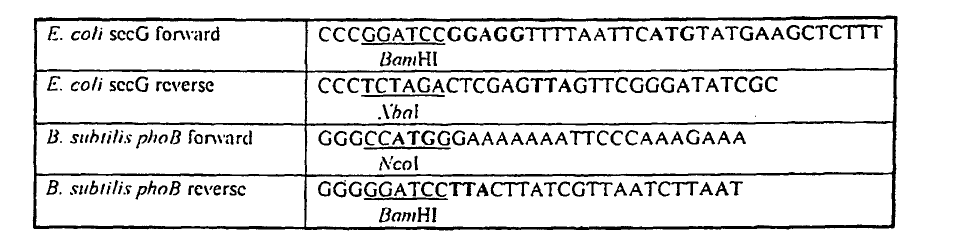

Recognition sites of restriction enzymes used are underlined. Ribosome-binding sites, and start and stop codons are indicated in bold.

**Table 2.**

| List of plasmids used in this study. | | | |
|---|---|---|---|
| name: | replicon | resistance | relevant expression |
| pDELG2 | ColE1 | Amp. Cam | - (deletion vector) |
| pPR111 | ColE1, repR | Amp. Phleo | - |
| pET302 | pBR | Amp | - |
| pET304 | pBR | Amp | *E. coli* SecG |
| pET324 | pBR | Amp | - |
| pET461 | pBR | Amp | *B. subtilis* PhoB (his-tagged) |
| pET470 | ColE1, repR | Amp. Phleo | *E. coli* SecG |
| pET471 | ColE1, repR | Amp. Phleo | *B subtilis* YvaL |
| pET468 | repA | Ery | α-amylase |
| pET472 | repA | Ery | α-amylase, *E. coli* SecG |
| pET473 | repA | Ery | α-amylase, *B. subtilis* YvaL |
| pET812 | pBR | Amp | *B. subtilis* SecYE |
| pET820 | pBR | Amp | *B. subtilis* YvaL |
| pET822 | pBR | Amp | *B. subtilis* SecYE-YvaL |

### c. Deletion of SecG from the chromosome of B.subtilis

Vector pDELG2 was digested with *Pvu*II to yield a 2.8 kb linear fragment containing the regions flanking the *yvaL*, which was replaced by a chloramphenicol resistance marker. *B. subtilis* DB104 was transformed with the fragment using natural competence (Young, 1967, Nature 213:773-775), and chloramphenicol resistant colonies were selected. The correct position of the chromosomal replacement was confirmed by PCR. In the resulting strain, DB104ΔG, the *yvaL* has been replaced by the chloramphenicol resistance gene while leaving the flanking regions intact.

### d. Growth experiments

*B. subtilis* DB104 and DB104ΔG were transformed with each of six plasmids constructed for testing, *i.e.* pPR111, pET470, pET471, pET468, pET472 and pET473. After transformation, plates were incubated at 30°C overnight. Selective pressure using the appropriate antibiotics was applied from this point onwards. No chloramphenicol was used at this stage. A single colony was picked for each transformant and cultured overnight at 30°C in liquid medium. 5µl of the overnight culture were struck on plates and incubated at temperatures ranging from 15°C to 30°C until the colonies of the wild-type strain reached a diameter of several millimetres. Plates were inspected daily and the occurrence and size of the colonies were noted.

For expression in *E. coli* plasmids pET820 and pET304 were transformed to *E. coli* KN370 (Δ*secG::kan*) as described before (Nishiyama et al., 1994, The EMBO Journal 13:3272-3277) and assay for the formation of single colonies on agar-plates at either 20°C or at 37°C, with or without induction using 1PTG (1mM).

### e. Analysis of secreted proteins

*B. subtilis* DB104 and DB104ΔG transformed with plasmid pET468 were grown overnight at 30°C in liquid medium. The cultures were cooled on ice and fractionated into a cellular fraction and culture medium by centrifugation. Alternatively, the overnight cultures were diluted 1:50 into fresh medium, grown to an OD₆₀₀ of 0.6 and incubated overnight at 15°C. The culture supernatant was precipitated with 10% w/v TCA, washed twice with cold acetone and analysed by SDS-PAGE. Cellular pellets of the cultures were resuspended in sample buffer, sonicated and analysed by SDS-PAGE. For further analysis of the cellular fractions, accessibility for proteinase K was tested. Transformed DB104 and DB104ΔG were grown overnight at 30°C and harvested by centrifugation. The cellular pellet was washed once with TN (50 mM TRIS-C1, pH 7.5, 100 mM NaC1) buffer, and resuspended in the same buffer containing 0.5 mg/ml lysozyme. After incubation for 15 min. on ice, proteinase K was added to a final concentration ranging from 0 to 2 mg/ml and the suspension was incubated for further 15 min. Finally, the suspension was precipitated with TCA, washed with acetone and analysed by SDS-PAGE.

### f. Expression of pET812 and pET822 and preparation of inside out vesicles

*E. coli* SF100 was used for the overexpression of *B. subtilis* SecY. SecE, and either SccG of *E. coli* (pET812) or YvaL of *B. subtilis* (pET822). Expression of the proteins and isolation of inside out vesicles was performed as described before (Van der Does et al., 1996).

### g. E.coli SecA stripping of the vesicles and in vitro translocation

To remove the *E. coli* SecA from the inside out vesicles 100 µl of vesicles (10 mg/ml) were incubated with 50 µl of polyclonal antibody directed against *E. coli* SecA (Schiebel et al., 1991, Molecular Microbiology 22: 619-629). *In vitro* translocation of ¹²⁵I-labeled his-prcPhoB (Van Wely et al., 1998, European Journal of Biochemistry) into inner membrane vesicles was assayed as described before (Cunningham, et al., 1989, Van Der Does et al., 1996) except that purified *B. subtilis* SecA (Van der Wolk et al., 1993, Molecular Microbiology 8:31-42) was used instead of *E. Coli* SecA (0.5 µg).

### h. Production of B. subtilis SecG polyclonal antibody

A peptide polyclonal antibody directed against the internal YvaL sequence Tyr-Ala-Glu-Gln-Leu-Phe-Gly-Lys-Gln-Lys-Ala-Arg-Gly-Leu-Asp coupled to KLH via the tyrosine residue was produced in Rabbits according to standard procedures by NEOSYSTEM, Strasbourg, France.

### Example II

This Example illustrates that B.subtilis SecG is a functional homolog of E.coli SecG.

The membrane vesicle derived from cells expressing pET812 and pET822 were stripped of their indigenous E.coli SecA using a polyclonal antibody directed against SecA and subjected to an in vitro translocation assay using ¹²⁵I-labeled his-prePhoB. In Figure 8, the result of the translocation is shown. When no B. subtilis SecA is added both vesicles containing either SecYEG or SecYE and YVAL show only little background translocation. However, when B.subtilis SecA is added to vesicles containing SecYE and YVAL, an enormous increase in translocation efficiency of ¹²⁵I-prePhoB is observed, while in the vesicles containing the SecYE and E.coli SecG no extra translocation is observed. From these data, it can be concluded that B.subtilis SecYE together with B.subtilis Yval and SecA forms a functional preprotein translocase that mediates the translocation of Bacillus prePhoB protein in vitro.

### Example III

This example illustrates that B.subtilis SecY, SecE and SecG (YVAL) proteins can be overexpressed in E.coli.

To establish whether the pET812 and pET 822 are expressed in E.coli SF100, inside out vesicles were analyzed on a 15% SDS-PAGE. Both the SecY and SecE of B.subtilis were readily visible on a commassie stained gel (Figure 7A). The B.subtilis SecG and increased amounts of E.coli SecG could be detected on an immunoblot using antibodies directed against these proteins (Figures 7B-7C).

### Example IV

This example illustrates the involvement of protein secretion machinery in the secretion of proteins for wild type cells and cells having a deletion in B.subtilis SecG.

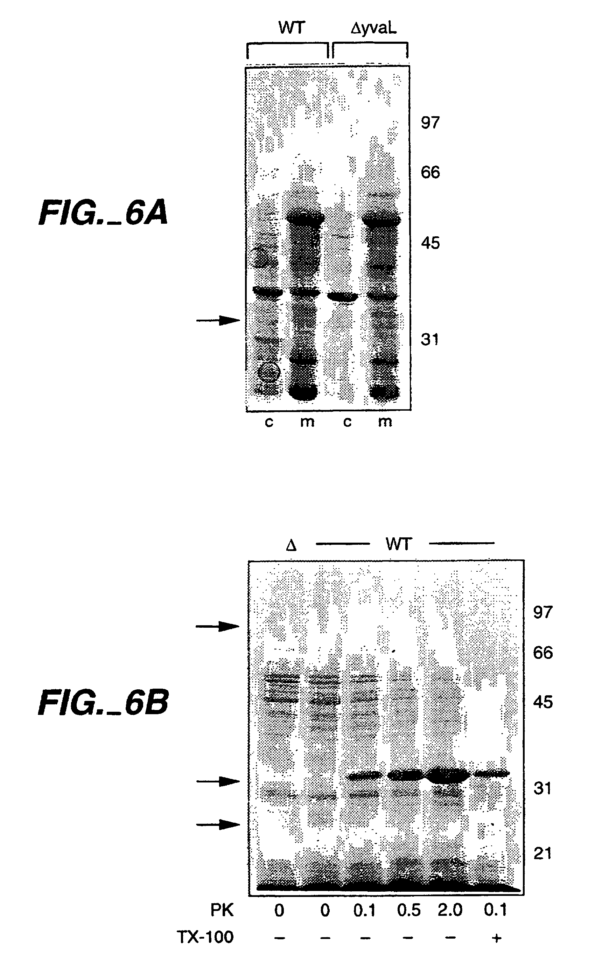

In the culture supernatants of cells grown at different temperatures, no differences between wild type and mutant cells was observed (Figure 6A). The cellular fraction, showed some differences in the banding pattern. The difference mainly concerns the absence of some bands in the mutant. The localization of these proteins was determined by breakdown of the cell wall by lysozyme and subsequent protease digestion of the accessible proteins (Figure 6B). Some of the protein bands are digested already by low concentrations of proteinase K, whereas breakdown of most other proteins only occurs after disruption of the cell membrane by Triton X-100. These proteins appear to be secreted. Some of these secreted proteins are absent in the mutant strain. Therefore, the B.subtilis SecG disruption mutant appears to be defective in the secretion of some extracellular proteins.

### Example V

This example illustrates the effect of a SecG deletion on cell growth.

Disruption of the E.coli secG gene has been shown to result in a cold-sensitive phenotype (Nishiyama et al., 1994, EMBO Journal 13:3272-3277), at non-permissive temperatures of 25 °C and below. Deletion of B.subtilis secG from the chromosome did not result in any phenotype when cells were grown at 37 °C either on rich or minimal media. Incubations below 20 °C demonstrated a mild cold sensitivity, where the DB104ΔG strain showed progressively slower growth as compared to DB104. The mutant strain didn't stop growing completely, however. Compared to the wild type, growth is retarded more severely when temperatures are lowered further. After shifting the cells again to higher temperatures, growth resumed at a faster rate.

Cells were transformed with plasmids expressing E.coli SecG or Bacillus subtilis SecG as well as a control plasmid. After preincubation at temperatures that do not affect growth of the mutant, cells were plated and incubated at several lower temperatures. Growth of the colonies was monitored over a period of several days. Wild type and mutant cells transformed with the control plasmid behaved like the non-transformed counterparts, showing retarded growth but not a complete stop at lower temperatures. Transformation of the mutant with pET471 expressing the secG gene product could relieve the retardation, showing that the phenotype of the mutant was not caused by any polar effects but by the deletion of secG itself. Surprisingly, when the mutant is transformed with pET470 expressing E.coli SecG, growth is stopped completely at temperatures of 20 °C or less. When the same plasmid is brought into the wild type cells, some interference with growth is observed at lower temperatures but not at 25 °C. A disruption of the secG gene renders Bacillus subtilis mild cold-sensitive, but is not an essential gene for B.subtilis. The results are presented in Table 3.

**Table 3.**

| Results of the growth experiments | | | | |
|---|---|---|---|---|
| strain: | expression | growth at: 15°C | 20°C | 25°C |
| DB104:: 111 | - | ++ | ++ | ++ |
| DB104 :: 470 | *E. coli* SecG | ± | ± | ++ |
| DB104 :: 471 | *B. sub* YvaL | ++ | ++ | ++ |
| ΔyvaL :: 111 | - | ± | ± | ++ |
| ΔyvaL :: 470 | *E. coli* SecG | - | - | ++ |
| ΔyvaL :: 471 | *B. sub* YvaL | ++ | ++ | ++ |
| DB104 :: 468 | α-amylase | ++ | ++ | ++ |
| DB104 :: 472 | α-amylase, *E. coli* SecG | ± | ± | ++ |
| DB104 :: 473 | α-amylase, *B. sub* YvaL | ++ | ++ | ++ |
| ΔyvaL :: 468 | α-amylase | - | - | ++ |
| ΔyvaL :: 472 | α-amylase, *E. coli* SecG | ± | ± | ± |
| ΔyvaL :: 473 | α-amylase, *B. sub* YvaL | ± | ± | ± |
| ++, growth like referenc; ±, growth, but slower than reference; -, no growth. | | | | |

### Example VI

This example illustrates the effect of expression of a secretory protein.

B. subtilis cells mutant in secG and wild type cells were transformed with plasmid pET468 and derivatives. These plasmids expression alpha-amylase thereby invoking secretory stress. Derivatives of pET472 and pET473 express alpha amylase in combination with E.coli SecG or B.subtilis SecG, respectively. Expression of alpha amylase did not retard growth of the deletion mutant at 30 °C, the temperature used for preculturing the cells. At this temperature, the halos that are formed by the alpha amylase on starch containing plates by transformants of wild type and deletion mutants are the same size. When pET468 transformants of the deletion mutant were shifted to lower temperatures, a clear and complete cold sensitivity was demonstrated. Already at 20 °C, cells stopped growing completely. When the cells were transformed back to the permissive temperature of 30 °C, after prolonged incubation at 20 °C, growth was not resumed. The deletion mutant is capable of sustaining a basic level of secretion even at lower temperatures, but cannot handle overexpression of a secreted protein over a broad temperature range.

### Example VII

### Detection of gram-postive microorganisms

The following example describes the detection of gram-positive microorganism SecG.

DNA derived from a gram-positive microorganism is prepared according to the methods disclosed in Current Protocols in Molecular Biology, Chap. 2 or 3. The nucleic acid is subjected to hybridization and/or PCR amplification with a probe or primer derived from SecG. A preferred probe comprises the nucleic acid section containing conserved amino acid sequences

The nucleic acid probe is labeled by combining 50 pmol of the nucleic acid and 250 mCi of [gamma ³²P] adenosine triphosphate (Amersham, Chicago IL) and T4 polynucleotide kinase (DuPont NEN®, Boston MA). The labeled probe is purified with Sephadex G-25 super fine resin column (Pharmacia). A portion containing 10⁷ counts per minute of each is used in a typical membrane based hybridization analysis of nucleic acid sample of either genomic or cDNA origin.

The DNA sample which has been subjected to restriction endonuclease digestion is fractionated on a 0.7 percent agarose gel and transferred to nylon membranes (Nytran Plus, Schleicher & Schuell, Durham NH). Hybridization is carried out for 16 hours at 40 degrees C. To remove nonspecific signals, blots are sequentially washed at room temperature under increasingly stringent conditions up to 0.1 x saline sodium citrate and 0.5% sodium dodecyl sulfate. The blots are exposed to film for several hours, the film developed and hybridization patterns are compared visually to detect polynucleotide homologs of *B.subtilis* SecG. The homologs are subjected to confirmatory nucleic acid sequencing. Methods for nucleic acid sequencing are well known in the art. Conventional enzymatic methods employ DNA polymerase Klenow fragment, SEQUENASE® (US Biochemical Corp, Cleveland, OH) or Taq polymerase to extend DNA chains from an oligonucleotide primer annealed to the DNA template of interest.

Various other examples and modifications of the foregoing description and examples will be apparent to a person skilled in the art after reading the disclosure without departing from the spirit and scope of the invention, and it is intended that all such examples or modifications be included within the scope of the appended claims. All publications and patents referenced herein are hereby incorporated in their entirety.

## Claims

1. An expression vector comprising nucleic acid encoding a gram-positive microorganism SecG which has at least 80% amino acid sequence identity to B. subtilis SecG set out in Figure 1 and is capable of modulating secretion in a gram-positive microorganism, wherein said secretion factor is under the control of expression signals capable of overexpressing said secretion factor in a gram-positive microorganism.

2. The expression vector of Claim 1 wherein said gram-positive microorganism is a *Bacillus*.

3. The expression vector of Claim 2 wherein the *Bacillus* is selected from the group consisting of *B. subtilis, B. licheniformis, B. lentus, B. brevis, B. stearothermophilus, B. alkalophilus, B. amyloliquefaciens, B. coagulans, B. circulans, B. lautus and Bacillus thuringiensis*.

4. A gram positive microorganism comprising the expression vector of Claim 1.

5. The microorganism of Claim 4 that is a *Bacillus.*

6. The microorganism of Claim 5 wherein the *Bacillus* is selected from the group consisting of *B. subtilis, B. licheniformis, B. lentus, B. brevis, B. stearothermophilus, B. alkalophilus, B. amyloliquefaciens, B. coagulans, B. circulans, B. lautus and Bacillus thuringiensis*.

7. The microorganism of Claim 4 wherein said microorganism is capable of expressing a heterologous protein.

8. The microorganism of Claim 7 wherein said heterologous protein is selected from the group consisting of hormone, enzyme, growth factor and cytokine.

9. The microorganism of Claim 8 wherein said heterologous protein is an enzyme.

10. The microorganism of Claim 9 wherein said enzyme is selected from the group consisting of a proteases, cellulases amylases, carbohydrases, lipases, reductases, isomerases, epimerases, tautomerases, transferases, kinases and phophatases.

11. An improved method for secreting a protein in a gram-positive microorganism comprising the steps of obtaining a gram-positive microorganism host cell transformed with nucleic acid encoding SecG which has at least 80% amino acid sequence identity to B. subtilis SecG set out in Figure 1 and is capable of modulating secretion in a gram-positive microorganism wherein said nucleic acid is under the control of expression signals capable of expressing SecG in a gram-positive microorganism and further comprising nucleic add encoding said protein; and culturing said microorganism under conditions suitable for expression of SecG and expression and secretion of said protein.

12. The method of Claim 11 wherein said gram-positive microorganism also comprises nucleic acid encoding at least one additional secretion factor selected from the group consisting of SecY, SecE and SecA.

13. The method of Claim 11 wherein said protein is homologous to said host cell.

14. The method of Claim 11 wherein said protein is heterologous to said host cell.

15. The method of Claim 11 wherein said gram-positive microorganism is a *Bacillus.*

16. The method of Claim 15 wherein said *Bacillus* is selected from the group consisting of *B. subtilis, B. licheniformis, B. lentus, B. brevis, B. stearothermophilus, B. alkalophilus, B. amyloliquefaciens, B. coagulans, B. circulans, B. lautus and Bacillus thuringiensis.*

17. The method of Claim 14 wherein said heterologous protein is selected from the group consisting of hormones, enzymes, growth factor, and cytokine.

18. The method of Claim 17 wherein said heterologous protein is an enzyme.

19. The method of Claim 18 wherein said enzyme is selected from the group consisting of a proteases, cellulases, amylases, carbohydrases, and lipases; isomerases such as racemases, epimerases, tautomerases, or mutases; transferases, kinases and phophatases.

## Patentansprüche

1. Expressionsvektor, umfassend eine Nukleinsäure, die den SecG von einem gram-positiven Mikroorganismus codiert, wobei sie mindestens 80 % Identität mit der Aminosäureseuqenz von B. subtilis SecG hat, die in Fig. 1 dargestellt ist, und die Sekretion in einen gram-positiven Mikroorganismus verändern kann, wobei der Sekretionsfaktor unter der Kontrolle der Expressionssignale steht, die diesen Sekretionsfaktor in einem gram-positiven Mikroorganismus überexprimieren können.

2. Expressionsvektor nach Anspruch 1, wobei der gram-positive Mikroorganismus Bacillus ist.

3. Expressionsvektor nach Anspruch 2, wobei der Bacillus ausgewählt wird aus der Gruppe, bestehend aus B. subtilis, B. licheniformis, B. lentus, B. brevis, B. stearothermophilus, B. alkalophilus, B. amyloliquefaciens, B. coagulans, B. circulans, B. lautus und Bacillus thuningiensis.

4. Gram-positiver Mikroorganismus, umfassend den Expressionsvektor von Anspruch 1.

5. Mikroorganismus nach Anspruch 4, der Bacillus ist.

6. Mikroorganismus nach Anspruch 5, wobei der Bacillus ausgewählt wird aus der Gruppe, bestehend aus B. subtilis, B. licheniformis, B. lentus, B. brevis, B. stearothermophilus, B. alkalophilus, B. amyloliquefaciens, B. coagulans, B. circulans, B. lautus und Bacillus thuningiensis.

7. Mikroorganismus nach Anspruch 4, wobei der Mikroorganismus ein heterologes Protein exprimieren kann.

8. Mikroorganismus nach Anspruch 7, wobei das heterologe Protein ausgewählt wird aus der Gruppe, bestehend aus einem Hormon, Enzym, Wachstumsfaktor und Cytokin.

9. Mikroorganismus nach Anspruch 8, wobei das heterologe Protein ein Enzym ist.

10. Mikroorganismus nach Anspruch 9, wobei das Enzym ausgewählt wird aus der Gruppe, bestehend aus Proteasen, Cellulasen, Amylasen, Carbohydrasen, Lipasen, Reduktasen, Isomerasen, Epimerasen, Tautomerasen, Transferasen, Kinasen und Phosphatasen.

11. Verbessertes Verfahren zum Sekretieren eines Proteins in einem gram-positiven Mikroorganismus, umfassend die Schritte: Erhalten eines gram-positiven Mikroorganismus als Wirtszelle, die mit einer Nukleinsäure transformiert ist, die SecG codiert, die mindestens 80 % Identität mit der Aminosäureseuqenz von B. subtilis SecG hat, die in Fig. 1 dargestellt ist, und die Sekretion in einen gram-positiven Mikroorganismus verändern kann, wobei die Nukleinsäure unter der Kontrolle der Expressionssignale steht, die SecG in einem gram-positiven Mikroorganismus exprimieren können und die ausserdem eine Nukleinsäure umfaßt, die das Protein codiert, und Kultivieren des Mikroorganismus unter Bedingungen, die sich zur Expression von SecG eignen, und Expression und Sekretion des Proteins.

12. Verfahren nach Anspruch 11, wobei der gram-positive Mikroorganismus ausserdem eine Nukleinsäure umfasst, die mindestens einen zusätzlichen Sekretionsfaktor, ausgewählt aus der Gruppe, bestehend aus SecY, SecE und SecA, codiert.

13. Verfahren nach Anspruch 11, wobei das Protein für die Wirtszelle homolog ist.

14. Verfahren nach Anspruch 11, wobei das Protein für die Wirtszelle heterolog ist.

15. Verfahren nach Anspruch 11, wobei der gram-positive Mikroorganismus Bacillus ist.

16. Verfahren nach Anspruch 15, wobei der Bacillus ausgewählt wird aus der Gruppe, bestehend aus B. subtilis, B. licheniformis, B. lentus, B. brevis, B. stearothermophilus, B. alkalophilus, B. amyloliquefaciens, B. coagulans, B. circulans, B. lautus und Bacillus thuningiensis.

17. Verfahren nach Anspruch 14, wobei das heterologe Protein ausgewählt wird aus der Gruppe, bestehend aus Hormonen, Enzymen, Wachstumsfaktor und Cytokin.

18. Verfahren nach Anspruch 17, wobei das heterologe Protein ein Enzym ist.

19. Verfahren nach Anspruch 18, wobei das Enzym ausgewählt wird aus der Gruppe, bestehend aus Proteasen, Cellulasen, Amylasen, Carbohydrasen und Lipasen, Isomerasen, wie Racemasen, Epimerasen, Tautomerasen oder Mutasen, Transferasen, Kinasen und Phosphatasen.

## Revendications

1. Vecteur d'expression comprenant un acide nucléique codant SecG de microorganisme gram-positif qui présente une identité de séquence d'acides aminés d'au moins 80 % par rapport à SecG de *B. subtilis*, établie sur la figure 1 et qui est apte à moduler la sécrétion dans un microorganisme gram-positif, dans lequel ledit facteur de sécrétion étant sous la commande de signaux d'expression aptes à sur-exprimer ledit facteur de sécrétion dans un microorganisme gram-positif.

2. Vecteur d'expression selon la revendication 1, dans lequel ledit microorganisme gram-positif est un *Bacillus*.

3. Vecteur d'expression selon la revendication 2, dans lequel le *Bacillus* est sélectionné dans le groupe comprenant *B. subtilis, B. licheniformis, B. lentus, B. brevis, B. stearothermophilus, B. alkalophilus, B. amyloliquefaciens, B. coagulans, B. circulans, B. lautus et Bacillus thuringiensis*.

4. Microorganisme gram-positif comprenant le vecteur d'expression de la revendication 1.

5. Microorganisme selon la revendication 4 qui est un *Bacillus.*

6. Microorganisme selon la revendication 5, dans lequel le *Bacillus* est sélectionné dans le groupe comprenant *B. subtilis, B. lichenformis, B. lentus, B. brevis, B. stearothermophilus, B. alkalophilus,* *B. amyloliquefaciens, B. coagulans, B. circulans, B. lautus et B. thuringiensis*.

7. Microorganisme selon la revendication 4, dans lequel ledit microorganisme est apte à exprimer une protéine hétérologue.

8. Microorganisme selon la revendication 7, dans lequel ladite protéine hétérologue est sélectionnée dans le groupe comprenant une hormone, une enzyme, un facteur de croissance et une cytokine.

9. Microorganisme selon la revendication 8, dans lequel ladite protéine hétérologue est une enzyme.

10. Microorganisme selon la revendication 8, dans lequel ladite enzyme est sélectionnée dans le groupe comprenant les protéases, les cellulases, les amylases, les carbohydrases, les lipases, les réductases, les isomérases, les épimérases, les tautomérases, les transférases, les kinases et les phosphatases.

11. Procédé amélioré de sécrétion d'une protéine dans un microorganisme gram-positif comprenant les étapes consistant à obtenir une cellule-hôte de microorganisme gram-positif transformée avec un acide nucléique codant SecG qui comporte une identité de séquence d'acides aminés d'au moins 80 % par rapport à SecG de *B. subtilis*, établie sur la figure 1 et qui est apte à moduler la sécrétion dans un microorganisme gram-positif, ledit acide nucléique étant sous la commande de signaux d'expression aptes à exprimer SecG dans un microorganisme gram-positif, ledit microorganisme comprenant en outre un acide nucléique codant ladite protéine ; et à cultiver ledit microorganisme dans des conditions appropriées à l'expression de SecG et à l'expression et la sécrétion de ladite protéine.

12. Procédé selon la revendication 11, dans lequel ledit microorganisme gram-positif comprend également un acide nucléique codant au moins un facteur de sécrétion additionnel sélectionné dans le groupe constitué de SecY, SecE et SecA.

13. Procédé selon la revendication 11, dans lequel ladite protéine est homologue à ladite cellule-hôte.

14. Procédé selon la revendication 11, dans lequel ladite protéine est hétérologue à ladite cellule-hôte.

15. Procédé selon la revendication 11, dans lequel ledit microorganisme gram-positif est un *Bacillus.*

16. Procédé selon la revendication 15, dans lequel ledit *Bacillus* est sélectionné dans le groupe comprenant *B. subtilis, B. licheniformis, B. lentus, B. brevis, B. stearothermophilus, B. alkalophilus, B. amyloliquefaciens, B. coagulans, B. circulans, B. lautus et B. thuringiensis*.

17. Procédé selon la revendication 14, dans lequel ladite protéine hétérologue est sélectionnée dans le groupe comprenant des hormones, des enzymes, le facteur de croissance et la cytokine.

18. Procédé selon la revendication 17, dans lequel ladite protéine hétérologue est une enzyme.

19. Procédé selon la revendication 18, dans lequel ladite enzyme est sélectionnée dans le groupe comprenant les protéases, les cellulases, les amylases, les carbohydrases, et les lipases, les isomérases, telles que les racémases, les épimérases, les tautomérases, ou les mutases, les transférases, les kinases et les phosphatases.
